# EUROPÄISCHE PATENTSCHRIFT

(11) **EP 1 216 981 B1**
(45) Veröffentlichungstag und Bekanntmachung des Hinweises auf die Patenterteilung: **02.08.2006**
(21) Anmeldenummer: 01126599.8
(22) Anmeldetag: 19.11.2001
(51) Int. Cl.: C07C 68/02

(54) **Kontinuierliches Verfahren zur Herstellung von Kohlensäurediarylestern**
Continuous process for the preparation of diaryl esters of carbonic acid
Procédé continu de préparation d'esters diaryliques d'acide carbonique

(30) Priorität: 19.12.2000 DE 10063297
(43) Veröffentlichungstag der Anmeldung: 26.06.2002
(73) Patentinhaber: Bayer MaterialScience AG, 51368 Leverkusen (DE)
(72) Erfinder: Alewelt, Wolfgang, Dr., 47809 Krefeld (DE); Kühling, Steffen, Dr., 40670 Meerbusch (DE); Vandem Eynde, Johan, 9052 Zwijnaarde (BE); Van Meirvenne, Dirk, Dr., 9100 Sint Niklaas (BE)

(56) Entgegenhaltungen:
- EP-A- 0 274 743
- US-A- 4 016 190
- US-A- 4 102 912
- US-A- 5 142 088

## Beschreibung

Gegenstand der vorliegenden Erfindung ist ein Verfahren zur Herstellung von Kohlensäurediarylestern in einem kontinuierlichen Zweistufenverfahren, in welchem die Kohlensäurediarylester durch Reaktion von Monophenolen und Phosgen in einem inerten Lösungsmittel in Gegenwart von Alkali in der Phasengrenzfläche bei niedrigen Reaktionstemperaturen hergestellt werden und der pH-Wert der Reaktion in jeder Stufe in einem engen Fenster gehalten wird.

Die Herstellung von Kohlensäurediarylestern durch den Phasengrenzflächenprozess ist prinzipiell in der Literatur, z.B. Chemistry and Physics of Polycarbonates, Polymer Reviews, H. Schnell, Vol. 9, John Wiley and Sons, Inc. (1964), S. 50/51, beschrieben. In US-P 4 016 190 wird ein Herstellungsverfahren von Kohlensäurediarylestern beschrieben, das bei Temperaturen >65°C betrieben wird. Der pH-Wert wird bei diesem Verfahren zunächst niedrig (pH 8 bis 9) und anschließend hoch (10 bis 11) eingestellt.

Bei diesen bekannten Verfahren lässt jedoch die Reinheit der direkt erhaltenen Produkte zu wünschen übrig, dies macht aufwendige Reinigungsoperationen erforderlich. Ein Problem stellen auch die hohen Restphenolwerte im Abwasser dieser Prozesse dar, welche die Umwelt belasten und die Klärwerke vor eine erhöhte Abwasserproblematik stellen. Zudem ist es bei Massenprodukten wie Diarylcarbonaten immer wünschenswert die Ausbeuten zu verbessern.

Es stellte sich daher, ausgehend vom oben geschilderten Stand der Technik die Aufgabe, ein Verfahren bereitzustellen, welches Produkte in hoher Reinheit und guter Ausbeute liefert, um den Reinigungsaufwand zu reduzieren. Auch eine Entlastung des Abwassers von organischer Fracht wäre wünschenswert, um die Umweltbelastung bzw. Abwasserproblematik in den Klärwerken zu verringern.

Es wurde nun überraschenderweise gefunden, dass bei der kontinuierlichen Herstellung von Kohlensäurediarylestern durch Reaktion von Monophenolen und Phosgen in einem inerten Lösungsmittel in Gegenwart von Alkali in der Phasengrenzfläche bei niedrigen Reaktionstemperaturen und einem pH-Wert der Reaktion in der ersten Stufe von 11-12 sowie einem pH-Wert der zweiten Stufe von 7,5-10,5 eine hohe Phosgenausbeute, ein hoher Umsatz und eine große Produktreinheit, bei gleichzeitig niedrigen Restphenolwerten im Abwasser erhalten werden. Dadurch können im Gegensatz zum Stand der Technik bereits direkt aus der Reaktion Produkte von hoher Reinheit erhalten werden. Dadurch wird der anschließende Reinigungsprozess stark vereinfacht.

Die niedrigen Restphenolwerte tragen zudem zu einer Verringerung der Umweltbelastung bzw. der Abwasserproblematik in den Klärwerken bei.

Das eingesetzte Alkali kann eine Alkalilauge (Na, K, Li, Ca-hydroxid) sein, bevorzugt ist Natronlauge, und wird im erfindungsgemäßen Verfahren vorzugsweise als 20-55 gew.-%ige, besonders bevorzugt 30-50 gew.-%ige Lösung eingesetzt.

Phosgen kann flüssig, gasförmig oder gelöst im inerten Lösungsmittel eingesetzt werden.

Geeignete Monophenole zum Einsatz in die Reaktion sind Phenole der Formel (I) 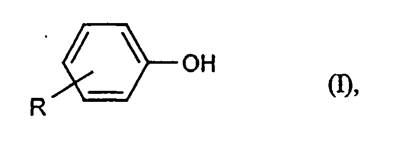 worin
- R: Wasserstoff, tert.-Butyl, Halogene oder ein verzweigter oder unverzweigter C₈- und/oder C₉-Alkylrest ist.

Somit also beispielsweise Phenol selbst, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol. Bevorzugt ist Phenol.

In dem Verfahren verwendete inerte organische Lösungsmittel sind beispielsweise Dichlormethan, Toluol, die verschiedenen Dichlorethane und Chlorpropanverbindungen, Chlorbenzol und Chlortoluol, vorzugsweise wird Dichlormethan eingesetzt.

Die Reaktionsführung wird kontinuierlich und bevorzugt in einer Pfropfenströmung ohne große Rückvermischung durchgeführt. Dies kann somit beispielsweise in Rohrreaktoren geschehen. Die Durchmischung der zwei Phasen (wässrige und organische Phase) kann durch eingebaute Rohrblenden, statische Mischer und/oder beispielsweise Pumpen realisiert werden.

In der ersten Stufe des erfindungsgemäßen kontinuierlichen Verfahrens wird die Reaktion der Reaktionskomponenten durch Zusammenbringen der Edukte Phosgen, des inerten Lösungsmittels, welches bevorzugt erst als Lösungsmittel für das Phosgen dient, und des Phenols, welches vorzugsweise zuvor bereits in der Alkalilauge gelöst ist, gestartet. Die Verweilzeit im kontinuierlichen erfindungsgemäßen Prozess, liegt in der ersten Stufe im Bereich von 2 Sekunden bis 300 Sekunden, bevorzugt im Bereich von 4 Sekunden bis 200 Sekunden. Der pH-Wert der ersten Stufe wird durch das Verhältnis von Alkalilauge/Phenol/Phosgen so eingestellt, dass der pH-Wert im Bereich von 11,0 bis 12,0, bevorzugt 11,2 bis 11,8, besonders bevorzugt bei 11,4 bis 11,6 liegt. Die Reaktionstemperatur der ersten Stufe wird durch Kühlung <40°C, bevorzugt <35°C gehalten.

In der zweiten Stufe des erfindungsgemäßen kontinuierlichen Verfahrens wird die Reaktion zum Kohlensäurediarylester komplettiert. Die Verweilzeiten liegen beim erfindungsgemäßen Prozess hierbei zwischen 1 Minute und 2 Stunden, bevorzugt zwischen 2 Minuten und 1 Stunde, ganz besonders bevorzugt zwischen 3 Minuten und 30 Minuten. In der zweiten Stufe des erfindungsgemäßen Verfahrens wird durch permanente Kontrolle des pH-Werts (wird im kontinuierlichen Verfahren bevorzugt Online nach den bekannten Verfahren gemessen) und entsprechende Einstellung des pH-Wertes durch Zugabe der Alkalilauge geregelt. Die Menge zugeführten Alkalilauge wird so eingestellt, dass der pH-Wert der zweiten Verfahrensstufe im Bereich von 7,5 bis 10,5, bevorzugt 8 bis 9,5, ganz besonders bevorzugt 8,2 bis 9,3 liegt. Die Reaktionstemperatur der zweiten Stufe wird durch Kühlung <50°C, bevorzugt <40°C, ganz besonders bevorzugt <35°C gehalten.

Die in dieser Anmeldung aufgeführten allgemeinen oder in Vorzugsbereichen aufgeführten Parameter bzw. Erläuterungen können jedoch auch untereinander, also zwischen den jeweiligen Bereichen und Vorzugsbereichen beliebig kombiniert werden.

Die wässrige Phase des erfindungsgemäßen Verfahrens weist dann weniger als 250 ppm, bevorzugt weniger 150 ppm, besonders bevorzugt weniger als 100 ppm Phenol, ganz besonders bevorzugt weniger als 50 ppm Phenol auf.

Im erfindungsgemäßen Verfahren wird Phosgen in bezug auf das Phenol im Verhältnis von 1,01 bis 1,15 Mol-%, bevorzugt 1,05 bis 1,12 Mol-% gefahren. Das Lösungsmittel wird so zugemischt, dass der Kohlensäurediphenylester in einer 5 bis 60 gew.-%igen Lösung, bevorzugt 20 bis 45 %igen Lösung nach der Reaktion vorliegt.

Die Reaktion kann durch Katalysatoren, wie tertiäre Amine, N-Alkylpiperidine oder Oniumsalze beschleunigt werden. Bevorzugt werden Tributylamin, Triethylamin und N-Ethylpiperidin verwendet. Die Konzentrationen der Katalysatoren sind 0,0001 mol bis 0,1 mol, bezogen auf das eingesetzte Phenol.

Unter Oniumsalzen werden in der vorliegenden Anmeldung Verbindungen wie NR4X, wobei R ein Alkyl und/oder Arylrest und/oder ein H sein kann und X ein Anion ist, verstanden.

Nach der Reaktion wird die organische, den Kohlensäurediarylester enthaltende Phase üblicherweise mit einer wässrigen Flüssigkeit gewaschen und nach jedem Waschvorgang von der wässrigen Phase soweit wie möglich getrennt. Die Wäsche erfolgt bevorzugt mit entsalztem Wasser. Die Kohlensäurediarylesterlösung ist nach der Wäsche und Abtrennung der Waschflüssigkeit üblicherweise trüb. Als Waschflüssigkeit werden wässrige Flüssigkeiten zur Abtrennung des Katalysators, z.B. eine verdünnte Mineralsäure wie HCl oder H₃PO₄, und zur weiteren Reinigung vollentsalztes Wasser eingesetzt. Die Konzentration von HCl oder H₃PO₄ in der Waschflüssigkeit kann beispielsweise 0,5 bis 1,0 Gew.-% betragen. Die organische Phase wird beispielhaft und vorzugsweise zweimal gewaschen.

Als Phasentrennvorrichtungen zur Abtrennung der Waschflüssigkeit von der organischen Phase können grundsätzlich bekannte Trenngefäße, Phasenseparatoren, Zentrifugen oder Coalescer oder auch Kombinationen dieser Einrichtungen verwendet werden.

Man erhält so ohne Berücksichtigung des noch abzutrennenden Lösungsmittels überraschend hohe Reinheitsgrade des Kohlensäurediarylesters von >99,85 %.

Zum Erhalt des hochreinen Kohlensäurediester wird das Lösungsmittel abgedampft. Das Abdampfen kann in mehreren Verdampferstufen erfolgen. Beispielsweise erfolgt dies durch eine oder mehrere hintereinandergeschaltete Destillationskolonnen bei der das Lösungsmittel vom Kohlensäurediarylester getrennt wird.

Diese Reinigungsstufe bzw. Stufen können beispielsweise kontinuierlich so geführt werden, dass die Sumpftemperatur bei der Destillation >150°C bis 310°C, bevorzugt >160 bis 230°C liegt. Die zur Durchführung dieser Destillation notwendigen Drücke liegen dabei zwischen 1 und 1000 mbar, bevorzugt zwischen 5 und 100 mbar.

Die so gereinigten Kohlensäurediester zeichnen sich durch besonders hohe Reinheit (GC >99,95%) und extrem gutes Umesterungsverhalten aus, so dass ein Polycarbonat in excellenter Qualität hergestellt werden kann.

Die Verwendung der Kohlensäurediarylester zur Herstellung von aromatischen Oligo-/Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder dem US-P 5 340 905 vorbeschrieben.

Die nachfolgenden Beispiele sollen die vorliegende Erfindung illustrieren ohne sie jedoch einzuschränken.

### Beispiele

### Beispiel 1

In einen senkrechtstehenden, gekühlten Rohrreaktor wird kontinuierlich ein Gemisch aus 103 kg/h VE-Wasser mit 42,2 kg/h 50 %iger NaOH und 48,3 kg/h Phenol mit einer Lösung von 86,2 kg/h Methylenchlorid und 27,5 kg/h Phosgen (8 Mol-% Überschuss bzgl. auf Phenol) zusammengebracht. Dieses Reaktionsgemisch wird auf eine Temperatur von 33°C gekühlt und nach einer mittleren Verweilzeit von 15 Sekunden ein pH-Wert von 11,5 gemessen. Zu diesem Reaktionsgemisch werden nun in der zweiten Stufe des Verfahrens 5,4 kg/h 50%iger NaOH zudosiert, so dass der pH-Wert der zweiten Reaktionsstufe nach einer weiteren Verweilzeit von 5 Minuten 8,5 beträgt. In der kontinuierlich betriebenen Reaktion werden auftretende Dosierschwankungen durch jeweilige Anpassungen der NaOH-Dosierungen aufgefangen. In der zweiten Stufe des Verfahrens wird das Reaktionsgemisch durch das Durchleiten eines mit Verengungen versehen Rohres ständig gemischt. Die Reaktionstemperatur wird nach erneuter Zugabe der NaOH durch Kühlen auf 30°C eingestellt. In der wässrigen schwach alkalischen Phase findet man 20 ppm Phenol. Dementsprechend hoch ist die DPC-Ausbeute bzgl. auf Phenol 99,998 %. Nach dem Abtrennen der organischen von der wässrigen Phase wird mit 0,6 %iger HCl und Wasser gewaschen und nach abschließender Phasentrennung nach dem Abdampfen des Methylenchlorids ein 99,9 %iges (GC-Methode) Diphenylcarbonat erhalten.

### Vergleichsbeispiel 1

Wie Beispiel 1, nur wird die Reaktionstemperatur der zweiten Stufe des Verfahrens nicht gekühlt, so dass die Temperatur 60°C beträgt. In der wässrigen schwach alkalischen Phase befinden sich dann 2400 ppm Phenol. Dementsprechend ist die DPC-Ausbeute bzgl. auf Phenol nur noch 99,76 %.

### Vergleichsbeispiel 2

Wie Beispiel 1, nur werden, anstatt der 5,4 kg/h, 7,8 kg/h 50 %iger NaOH zudosiert, so dass der pH-Wert der zweiten Reaktionsstufe nach einer weiteren Verweilzeit von 5 Minuten 11,7 beträgt. In der wässrigen schwach alkalischen Phase befinden sich dann 900 ppm Phenol. Dementsprechend ist die DPC-Ausbeute bzgl. auf Phenol nur noch 99,91 %.

## Patentansprüche

1. Verfahren zur kontinuierlichen Herstellung von Kohlensäurediarylestern in zwei Verfahrensstufen durch Reaktion von Monophenolen und Phosgen in einem inerten Lösungsmittel in Gegenwart von Alkali in der Phasengrenzfläche, **dadurch gekennzeichnet, dass** in der ersten Verfahrensstufe der pH-Wert in einem Fenster von 11-12, die Temperatur <40°C und in der zweiten Verfahrensstufe der pH in einem Fenster von 7,5-10,5, die Temperatur <50°C gehalten wird.

2. Verfahren gemäß Anspruch 1, **dadurch gekennzeichnet, dass** in der ersten Verfahrensstufe ein pH-Fenster von 11,2 bis 11,8 und in der zweiten Stufe ein Fenster von 8 bis 9.5 eingehalten wird.

3. Verfahren gemäß Anspruch 1, **dadurch gekennzeichnet, dass** in der ersten Stufe ein pH-Fenster von 11,4 bis 11,6 und in der zweiten Stufe ein Fenster von 8,2 bis 9,3 eingehalten wird.

4. Verfahren gemäß Anspruch 1, **dadurch gekennzeichnet, dass** in der ersten Stufe die Temperatur < 35°C und in der zweiten Stufe < 40°C gehalten wird.

5. Verfahren gemäß Anspruch 1, **dadurch gekennzeichnet, dass** in der zweiten Stufe die Temperatur < 35°C gehalten wird.

## Claims

1. Process for the continuous production of carboxylic acid diaryl esters in two process steps by the reaction of monophenols and phosgene in an inert solvent in the presence of alkali and at the phase boundary, **characterised in that** in the first process step the pH is maintained within a window of 11-12 and the temperature is maintained at <40°C, and in the second process step the pH is maintained within a window of 7.5-10.5 and the temperature is maintained at <50°C.

2. Process according to claim 1, **characterised in that** in the first process step a pH window of 11.2 to 11.8 is maintained and in the second step a window of 8 to 9.5 is maintained.

3. Process according to claim 1, **characterised in that** in the first step a pH window of 11.4 to 11.6 is maintained and in the second step a window of 8.2 to 9.3 is maintained.

4. Process according to claim 1, **characterised in that** in the first step the temperature is maintained at <35°C and in the second step it is maintained at <40°C.

5. Process according to claim 1, **characterised in that** in the second step the temperature is maintained at <35°C.

## Revendications

1. Procédé de préparation continue d'esters diaryliques d'acide carbonique en deux étapes, par réaction de monophénols et de phosgène dans un solvant inerte, en présence d'alcalis dans l'interface, **caractérisé en ce que** dans la première étape, le pH est maintenu dans une fenêtre de 11-12 et la température est maintenue en deçà de 40 °C et que dans la deuxième étape, le pH est maintenu dans une fenêtre de 7,5-10,5 et la température est maintenue en deçà de 50 °C.

2. Procédé selon la revendication 1, **caractérisé en ce que** dans la première étape, une fenêtre de pH de 11,2 à 11,8 et dans la deuxième étape, une fenêtre de 8 à 9,5 sont respectées.

3. Procédé selon la revendication 1, **caractérisé en ce que** dans la première étape, une fenêtre de pH de 11,4 à 11,6 et dans la deuxième étape, une fenêtre de 8,2 à 9,3 sont respectées.

4. Procédé selon la revendication 1, **caractérisé en ce que** la température est maintenue en deçà de 35 °C dans la première étape, et en deçà de 40 °C dans la deuxième étape,

5. Procédé selon la revendication 1, **caractérisé en ce que** dans la deuxième étape, la température est maintenue en deçà de 35 °C.
